# EUROPEAN PATENT APPLICATION

(11) **EP 4 527 943 A1**
(43) Date of publication of application: **26.03.2025**
(21) Application number: 23824282.0
(22) Date of filing: 16.06.2023
(51) Int. Cl.: C12Q 1/6876, C12Q 1/70, C12N 9/22, C12N 15/113

(54) **DIAGNOSTIC MICROPARTICLE PROBE COMPRISING MAGNETIC PARTICLE AND INACTIVATED GENETIC SCISSORS, AND MULTI-DIAGNOSTIC SYSTEM AND METHOD INVOLVING PROBE**

(30) Priority: 16.06.2022 KR 20220073702
(71) Applicant: Ezdiatech Inc., Chungcheongnam-do 31116 (KR)
(72) Inventor: JUNG, Yong-Gyun, Seoul 05792 (KR); CHOI, Young Wook, Seoul 01909 (KR); CHOI, Heelak, Ansan-si Gyeonggi-do 15345 (KR)
(74) Representative: Prüfer & Partner mbB Patentanwälte · Rechtsanwälte
(86) International application number: PCT/KR2023/008357
(87) International publication number: WO 2023/244069

(57) **Abstract**

The present invention relates to a diagnostic microparticle probe comprising a magnetic particle and an inactivated genetic scissors, and a multi-diagnostic system and multi-diagnostic method comprising the same, which may not only perform a rapid and accurate diagnosis compared to conventional diagnostic methods but also may be used for multiple diagnoses, by introducing genetic scissors technology into a diagnostic microparticle probe.

## Description

### [Technical Field]

The present invention relates to a diagnostic microparticle probe comprising a magnetic particle and an inactivated genetic scissors, and a multi-diagnostic system and multi-diagnostic method comprising the same, and more specifically, to a diagnostic microparticle probe comprising a magnetic particle and an inactivated genetic scissors, and a multi-diagnostic system and multi-diagnostic method comprising the same, which may not only perform a rapid and accurate diagnosis compared to conventional diagnostic methods but also may be used for multiple diagnoses, by introducing genetic scissors technology to a diagnostic microparticle probe.

### [Background Art]

*In vitro* diagnosis (IVD) is a technology that allows diagnosis of health conditions by analyzing objects such as blood, urine, and cells collected from the human body, and includes immunodiagnostics, self-monitoring of blood glucose, molecular diagnostic technologies, etc. Among these, molecular diagnostic technologies are technologies that utilize molecular biological techniques to diagnose diseases, and their scope is gradually expanding due to the emergence of newly discovered genetic mutations and infectious diseases in the fields of hereditary diseases and infectious diseases.

Among the molecular biological techniques, the recently developed genetic scissors technology is a technology that recognizes specific base sequences of genes in cells and edits them as desired, and has received attention as an innovative technology that can treat genetic diseases. The genetic scissors technology has been continuously developing (1st generation: zinc finger nuclease; 2nd generation: transcription activator-like effector nucleases (TALENs); 3rd generation: CRISPR-Cas), and recent efforts to develop genetic scissors technology beyond "gene editing" into a more rapid and more accurate molecular diagnostic technology are attracting attention.

Meanwhile, due to the current global pandemic situation, as of May 2022, COVID-19 has infected 520 million people around the world and killed 6.3 million people, and even in Korea, 18 million people have been infected and 24,000 people have died, surpassing the record of the Spanish flu, which is considered the worst pandemic of the 20th century. Rapid diagnosis is necessary to manage this pandemic situation, but since diagnosis takes about 1 to 2 days in Korea and usually takes about 4 to 7 days in the United States, it is impossible to respond effectively to reduce the spread of the disease. In addition, in the process of performing conventional molecular diagnosis, partial binding or repeated binding of markers during the reaction process causes amplification of nucleic acids, and the sequence that acts as a probe becomes positive, resulting in a false positive result. Therefore, there is an urgent need for new point-of-care testing technologies capable of providing accurate diagnostic results within 30 to 40 minutes.

### [Disclosure]

### [Technical Problem]

In order to solve the above-mentioned problems, the present invention aims to provide a diagnostic microparticle probe comprising a magnetic particle and an inactivated genetic scissors, and a multi-diagnostic system and multi-diagnostic method comprising the same, which may not only perform a rapid and accurate diagnosis compared to conventional diagnostic methods but also may be used for multiple diagnoses, by introducing genetic scissors technology to a diagnostic microparticle probe.

### [Technical Solution]

To solve the above-mentioned problems, the present invention provides a diagnostic microparticle probe comprising a microparticle; a capture probe introduced to the surface of the microparticle; and an inactivated genetic scissors bound to the capture probe and containing a gene sequence complementary to a target nucleic acid.

In one embodiment, the inactivated genetic scissors may comprise a guide RNA.

In one embodiment, the guide RNA may have a nucleotide sequence capable of hybridizing with a target site of the target nucleic acid gene.

In one embodiment, the inactivated genetic scissors may have an inactivated target nucleic acid cleavage function.

In one embodiment, the microparticle may contain magnetic particles therein.

In one embodiment, the microparticle may have a core-shell structure comprising a core containing a magnetic material and a shell layer surrounding the core and having a uniform thickness.

In one embodiment, the microparticle may have a size and specific gravity that prevents it from floating in water.

The present invention also provides a multi-diagnostic system comprising the diagnostic microparticle probe; a reporter nucleic acid complementarily bound to a target nucleic acid and labeled with biotin; and a reagent for nucleic acid amplification.

In one embodiment, the diagnostic microparticle probe may be labeled to be distinguished from each other by diameter, thickness, length, shape or identification code.

In one embodiment, the diagnostic microparticle probe may be a mixture of two or more diagnostic microparticle probes with different lengths.

In one embodiment, the reporter nucleic acid may be a mixture of two or more reporter nucleic acids to which phosphors with different colors are bound.

The present invention also provides a method for detecting a target nucleic acid, comprising the steps of: (a) extracting a target nucleic acid from a sample; (b) amplifying the target nucleic acid and binding a reporter nucleic acid thereto; (c) providing a diagnostic microparticle probe comprising a microparticle; a capture probe introduced to the surface of the microparticle; and an inactivated genetic scissors bound to the capture probe and containing a gene sequence complementary to the target nucleic acid; (d) reacting the diagnostic microparticle probe with the target nucleic acid to capture the target nucleic acid and the reporter nucleic acid; (e) attaching a fluorescent material to the reporter nucleic acid; and (f) detecting the target nucleic acid by measuring a fluorescent signal emitted from the dyed reporter nucleic acid.

In one embodiment, the detecting of the target nucleic acid may be multi-detecting two or more of the target nucleic acids using two or more of the diagnostic microparticle probes.

In one embodiment, the multi-detecting may comprise the process of reading the diagnostic microparticle probes labeled to be distinguished from each other by length, diameter, thickness, shape, color or identification code.

In one embodiment, the method may further comprise washing the diagnostic microparticle probe to which the target nucleic acid and reporter nucleic acid are attached, after the step (e).

In one embodiment, the method for detecting the target nucleic acid may be performed on a microwell.

In one embodiment, the diagnostic microparticle probe may be moved and immersed between the microwells by an external magnetic force.

### [Advantageous Effects]

The diagnostic microparticle probe according to the present invention, and the multi-diagnostic system and the multi-diagnostic method comprising the same make multi-diagnosis easier and enables rapid diagnosis than conventional methods by excluding non-specific secondary cleavage enzyme activity, and therefore, the use of the detection method according to the present invention is more effective for point-of-care testing (POCT) multi-diagnosis.

In addition, the diagnostic microparticle probe according to the present invention, and the multi-diagnostic system and the multi-diagnostic method comprising the same may detect target nucleic acids at a faster rate than conventional diagnostic methods, and therefore, they may be usefully used in the diagnosis of infections caused by viruses or bacteria.

### [Description of Drawings]

FIG. 1 shows the structure of a molecular diagnostic microparticle probe according to one embodiment of the present invention and its binding relationship with a target nucleic acid, a reporter nucleic acid and a fluorescent molecule.
FIG. 2 is a drawing showing one embodiment of a diagnostic microparticle probe according to one embodiment of the present invention.
FIG. 3 is a drawing showing a process for detecting a target nucleic acid using a magnetic microparticle/inactivated genetic scissors technology according to one embodiment of the present invention.
FIG. 4 is a drawing showing a process for detecting a target nucleic acid using a magnetic microparticle/inactivated genetic scissors technology according to one embodiment of the present invention, wherein A represents the process with gene amplification, and B represents the process without gene amplification.
FIG. 5 is a drawing showing in detail the process of capturing a target gene in the process of FIG. 3 according to one embodiment of the present invention, wherein A represents the process with gene amplification, and B represents the process without gene amplification.
FIG. 6 virtually shows diagnostic results by the process according to one embodiment of the present invention.
FIG. 7 shows the results of preliminarily confirming the possibility of capturing nucleic acid from a magnetic particle-based target sample.
FIG. 8 shows a capture and amplification process of a target nucleic acid sequentially performed from Well 1 to Well 4 according to one embodiment of the present invention.
FIG. 9 shows the result of performing a capture and amplification process of a nucleic acid for influenza B virus RNA according to one embodiment of the present invention.
FIG. 10 shows the result of introducing amplified DNA according to one embodiment of the present invention to the multi-diagnostic system of the present invention.
FIG. 11 shows the results of detecting each virus using a multi-diagnostic method according to one embodiment of the present invention.
FIG. 12 shows the results of simultaneously detecting each virus using a multi-diagnostic method according to one embodiment of the present invention.

### [Best Mode]

Hereinafter, preferred embodiments of the present invention will be described in detail. In describing the present invention, if it is determined that a specific description of related known technologies may obscure the gist of the present invention, the detailed description thereof will be omitted. Throughout the specification, it is to be understood that the singular forms comprise plural referents unless the context clearly dictates otherwise, and it is to be understood that the terms such as "comprise" or "have" as used in the present specification are intended to designate the presence of stated features, numbers, steps, operations, components, parts or combinations thereof, but not to preclude the possibility of the presence or addition of one or more other features, numbers, steps, operations, components, parts or combinations thereof. In addition, in performing the method or preparation method, each process constituting the method may occur in a different order from the specified order unless a specific order is clearly described in context. That is, each process may occur in the same order as specified, may be performed substantially simultaneously, or may be performed in the reverse order.

The technology disclosed in this specification is not limited to the embodiments described herein and may be embodied in other forms. However, the embodiments introduced herein are provided so that the content disclosed herein may be thorough and complete, and the technical spirit of the present technology may be sufficiently understood by those skilled in the art. In the drawings, in order to clearly express the components of each device, the size of the components, such as width or thickness, is shown somewhat enlarged. Overall, when describing the drawings, it was described from the observer's point of view, and when one element is referred to as being located on another element, this comprises all meanings that one element may be located directly on another element or additional elements may be interposed between them. In addition, those skilled in the art will be able to implement the spirit of the present invention in various other forms within the scope that does not depart from the technical spirit of the present invention. In addition, the same reference numerals on a plurality of drawings refer to elements that are substantially the same as each other.

In this specification, the term 'and/or' comprises a combination of a plurality of recited items or any one of a plurality of recited items. In this specification, 'A or B' may comprise 'A,' 'B,' or 'both A and B'.

The present invention relates to a diagnostic microparticle probe comprising a microparticle; a capture probe introduced to the surface of the microparticle; and an inactivated genetic scissors bound to the capture probe and containing a gene sequence complementary to the target nucleic acid.

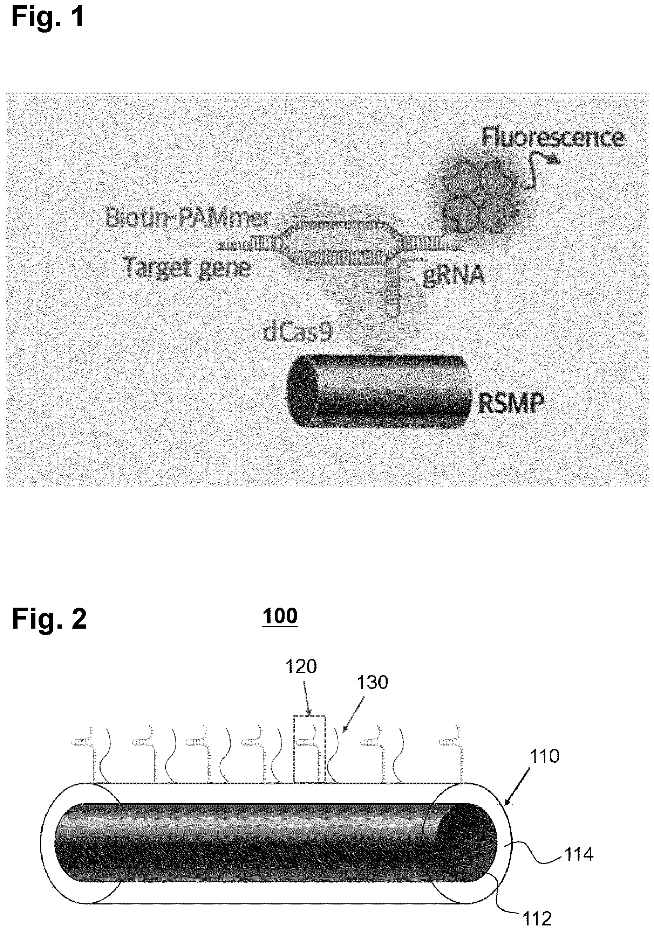

Hereinafter, the diagnostic microparticle probe according to the present invention will be described with reference to FIGS. 1 and 2.

The microparticle 110 may have a single structure or a core-shell structure. Preferably, the microparticle 110 may have a core-shell structure of a core 112 and a protective shell 114 surrounding the core 112. The core 112 of the microparticle 110 may include a magnetic material, for example, a magnetic responsive metal, wherein the magnetic responsive metal may be a paramagnetic material. Specifically, the magnetic responsive metal may be an alloy containing iron (Fe), nickel (Ni), cobalt (Co) or manganese (Mn). The magnetic responsive metal may contain transition metals such as iron, nickel, cobalt and manganese as main components, and rare earth metals such as gadolinium (Gd), terbium (Tb) and samarium (Sm), and may contain other elements such as boron (B), silicon (Si) and carbon (C). Representative examples of the magnetic responsive metals may be iron alloys or cobalt alloys. A specific example of the iron alloy may be Fe₇₀B₁₅Si₁₀C₅, and an example of the cobalt alloy may be Co₆₈Mn₇Si₁₀B₁₅.

Preferably, the microparticle 110 has a size and specific gravity that prevents it from floating on water so that it can be quickly collected or separated by an external magnet such as a permanent magnet or an electromagnet. In this case, the core 112 may occupy 60% or more of the total volume of the microparticle 110. For example, the volume of the core 112 may be 60 to 99%, preferably 75 to 99%, of the total volume of the microparticle 110. If the volume of the core is less than 60%, the magnetism of the core may be reduced, thereby making it difficult to move using a magnetic rod, and if it excesses 99%, the thickness of the shell may be reduced, thereby resulting in reduced durability.

In addition, the microparticle 110 may have a size (e.g., diameter or length) of tens to hundreds of µm, and preferably has a specific gravity of 5 or more. If the microparticle 110 is a nanoparticle with a size of less than 1 micrometer, it may float in water even if it is a particle with a high specific gravity (e.g., 7.876 for iron). In this case, in the process of analyzing single base polymorphism using microparticles 110, the magnetic control of the microparticles may not be smooth due to the low magnetic force of the microparticles when a magnetic field is applied, which may result in difficulty in separation. When the magnetic rod is moved up and down within the wells to promote an immune response, detachment and attachment of microparticles occurs, and as the magnetic rod moves up and down, the microparticles once attached to the magnetic rod do not fall back to the bottom of the wells due to their low weight even when the magnetic field is removed, and may continue to remain on the magnetic rod through non-specific binding. In this case, the reproducibility of quantitative analysis may be reduced when biomaterials within wells are detected.

The diagnostic microparticle probe according to one embodiment of the present invention reacts sensitively to magnetic force because its size is much larger than that of conventional silica beads and the magnetic responsive metal (magnetic core) occupies most of the volume of the microparticle unlike conventional silica beads in which magnetic particles are usually dispersed inside the silica, and thus has excellent reproducibility in quantitative analysis.

Meanwhile, the diagnostic microparticle probe 100 of the present invention forms a core-shell structure by having a magnetic responsive metal at the center and a shell layer surrounding it, wherein the shell layer 114 may consist of an organic or inorganic material and is preferably glass. In addition, a capture probe such as an antibody, a protein, a nucleic acid and a metabolite may be fixed to the surface of the diagnostic microparticle probe 100. Preferably, for this purpose, a functional group such as an acrylic group, a hydroxyl group, an amine group and a carboxyl group may be introduced to the surface.

The shell layer 114 may substantially completely surround the microparticle 110, but when necessary or in the manufacturing process, the surface of the microparticle may not be completely covered by the shell layer and some areas of the surface of the microparticle may be exposed.

The shell layer 114 may have a thickness of 1 to 100 µm, preferably 1 to 50 µm, more preferably 1 to 10 µm, and even more preferably 4 to 8 µm. If the shell layer 114 has a thickness less than the range, the surface of the shell layer 114 may be easily broken or cracked, and if it has a thickness exceeding the range, problems may occur depending on the laser characteristics and wavelength during glass cutting processing.

The core-shell structure may be formed by applying a liquid shell component to a core metal or by filling a core metal component into a hollow frame.

In this case, the shell layer 114 may be solidified from a liquid coating solution of an organic or inorganic material. More specifically, the shell layer 114 may be formed by preparing a liquid coating solution in such a way that a shell component in the form of an organic or inorganic material melts or becomes flowable at a high temperature or in such a way that it is dissolved in a solvent, and then applying the liquid coating solution to the core metal. The organic material may be mainly a polymer, and the inorganic material may be a metal or ceramic, particularly glass. For example, to form a shell layer 114, a coating solution obtained by dissolving plastic in a solvent or melting glass may be applied to the microparticle 110 by dip coating, spray coating, etc.

For example, when a glass tube is used as the hollow frame, there are a method of drawing a glass tube while injecting metal powder into the glass tube and then melting the metal at a high temperature, a method of injecting molten metal into a glass material while first drawing the glass material, a method of filling a glass tube with a dispersion obtained by dispersing metal powder in an ultraviolet-curable material, and then irradiating ultraviolet rays to cure it, etc. In these methods, the hollow frame itself may become the shell layer.

The microparticle 110 obtained by the above-described method have excellent surface uniformity. Conventional bioassay particles are grown on the surface of core particles using a silica precursor such as TEOS to form a shell layer 114, wherein the shell layer 114 has a very rough surface. Thus, non-specific binding of the material to be detected may occur frequently. This may cause unnecessary background noise.

On the other hand, glass forming the shell layer 114, for example, borosilicate glass, may minimize non-specific binding due to adsorption by chemical reaction with the reaction sample. In particular, the microparticle 110 according to one embodiment of the present invention has a coating layer derived from a liquid component as a shell layer 114, and therefore has a very uniform surface. The surface of the shell layer 114 may have an average surface roughness (Ra) of 15 nm or less, preferably 10 nm or less, more preferably 5 nm or less, even more preferably 2 nm or less, and particularly 1.5 nm or less. In addition, Ra may be, for example, 3 nm or more, 2 nm or more, or 1 nm or more. If the surface roughness is within the range, non-specific adsorption of the reaction sample on the surface of the shell layer 114 may be minimized.

Considering strength and transparency, the shell layer 114 of the microparticle 110 may be made of glass. The glass may contain a compound selected from the group consisting of soda lime, borosilicate, aluminosilicate, silica, alkali silicate, Pyrex and quartz as a main component. Preferably, for experimental purposes where heat resistance, acid resistance and water resistance are required, the glass may be borosilicate.

The microparticle 110 may have various shapes, including regular shapes such as rods, flat plates, spheres, etc., or irregular shapes. Even when the microparticle 110 has a flat plate shape, the cross-section may have various shapes such as a star, polygon, circle, etc., and is not particularly limited. Preferably, the microparticle is preferably in the form of a microrod, microdisc or microbead for convenience of manufacture and ease of observation, and is particularly preferably in the form of a microrod. If the microparticles 110 have the form of a microrod, it is easy to distinguish between overlapping microparticles, and if they are placed within a well, focusing is easy, and the area occupied by individual particles is small, so that a large number of microparticles may be observed on a single observation screen. Meanwhile, if the microparticles have a shape with a complex cross-sectional structure, such as a star shape, breakage at the edges may occur due to collisions with each other or the walls while moving inside the well, so that it is more preferable to have a simple shape such as a microrod.

The microrod may have a length of 10 to 1,000 µm. If the length of the microrod is less than the range, it is not easy to distinguish between particles of different lengths, and if it exceeds the range, the particles may overlap, so that observation may not be easy. In addition, the microrod may have a length to diameter ratio (aspect ratio) of 2 or more, 5 or more, or 10 or more. The upper limit of the aspect ratio may be 20 or less, 10 or less, or 5 or less. If the aspect ratio is less than the range, it resembles spherical particles, so that it may be difficult to distinguish each other, and if it is too large, it may be warped.

In a preferred embodiment, the microparticle 110 may be a cut piece of a glass-coated metal microwire. Microparticles 110 with various lengths may be obtained by simply cutting glass-coated metal microwires with a laser.

The above-described microparticles may be suitably used for diagnosis in a specific sample derived from a living organism. The samples may be tissue extracts, cell lysates, whole blood, plasma, serum, saliva, ocular fluid, cerebrospinal fluid, sweat, urine, milk, ascites fluid, synovial fluid, peritoneal fluid, etc. For rapid diagnosis, the pretreatment of the sample solution may be simplified or, in some cases, omitted.

For POCT diagnosis in a specific sample within a sample derived from a living organism, the microparticles may be prepared and used as particles of different sizes, lengths or shapes.

The capture probe 120 may be introduced to the microparticle 110 to capture a biomarker derived from a sample, particularly a nucleic acid-based biomarker. The biomarker is available without limitation as long as it is used in conventional scientific or medical fields, such as measuring or evaluating biological treatment processes, processes causing pathogenicity and pharmacological processes for treatment. The biomarker may be, for example, a polypeptide, peptide, nucleic acid, protein or metabolite that may be detected in biological fluids such as blood, saliva and urine, and preferably, the biomarker is a nucleic acid in that a biomarker associated with a specific disease may be detected with high sensitivity and specificity.

In order to capture a target nucleic acid extracted from a sample, the microparticle according to one embodiment of the present invention may have a capture probe 120 introduced onto the shell layer 114. The capture probe 120 is complementary to the target nucleic acid, and thus acts to specifically bind thereto to fix the target nucleic acid to the microparticle 110.

The capture probe may comprise an inactivated genetic scissors comprising a genetic sequence complementary to a target nucleic acid.

General genetic scissors bind to a target nucleic acid and then cleave a part of the target nucleic acid by an external stimulus. This behavior appears to cleave the desired location of the gene like scissors, and thus is called genetic scissors or CRISPR scissors. The present invention focuses on the binding ability of such genetic scissors to a target nucleic acid and uses the genetic scissors for binding to the target nucleic acid, but may maintain the sequence of the target nucleic acid while binding to it by inactivating the target nucleic acid cleavage function of the genetic scissors.

In this case, the genetic scissors may inactivate the target nucleic acid cleavage function by a conventional method. For example, the nucleic acid cleavage function of the genetic scissors may be selectively inactivated by heat treatment, acid or base treatment, ultraviolet irradiation, insertion of a heterologous base sequence, etc.

The inactivated genetic scissors may comprise a guide RNA. The guide RNA refers to RNA specific to a target nucleic acid (e.g., RNA capable of hybridizing with a target site of DNA) and may be used as an element of the CRISPR system together with a cleavage enzyme. However, since the cleavage function of the CRISPR system (genetic scissors) in the present invention is inactivated, the cleavage enzyme may not be included. In general, when the guide RNA selectively binds to the target nucleic acid and then the cleavage enzyme is introduced, the target nucleic acid is cleaved, but in the present invention, since this cleavage function is inactivated, the guide RNA may only be used for selective binding to the target nucleic acid.

The capture probe 120 may be fixed by adsorbing or chemically linking it to the surface of the microparticle 110, and preferably may be linked to a functional group on the surface of the shell layer 114. For example, the capture probe 120 may be attached to the microparticle 110 by introducing biotin to the surface of the capture probe 120 and introducing avidin, neutravidin or strepavidin that binds to biotin to the microparticle 110. Alternatively, the capture probe 120 may be linked to the microparticle 110 using a hydroxyl group, amino group, or carboxyl group on the surface of the microparticle 110.

The present invention also provides a POCT multi-diagnostic system comprising the diagnostic microparticle probe; a reporter nucleic acid complementarily bound to a target nucleic acid and labeled with biotin; and a reagent for nucleic acid amplification.

As for the diagnostic microparticle probe, it is omitted because it is the same as described above.

The reporter nucleic acid refers to a nucleic acid that generates a signal depending on the presence or absence of a desired target nucleic acid, and the reporter nucleic acid of the present invention may complementarily bind to the target nucleic acid and may be labeled with biotin.

As described above, the biotin-labeled reporter nucleic acid may complementarily bind to part or all of the target nucleic acid, and in particular, may be amplified simultaneously during the amplification process of the target nucleic acid and complementarily bind to the target nucleic acid. Through this, most of the target nucleic acids exist bound to the biotin-labeled reporter nucleic acids, which means that most of the target nucleic acids bound to the diagnostic microparticle probe are bound to the biotin-labeled reporter nucleic acids.

In the diagnostic method using conventional microparticles, the reporter nucleic acid was provided as part of a probe bound to the microparticles. That is, the reporter nucleic acid exists as a part of the diagnostic microparticle probe, and generates a signal by controlling whether or not this reporter nucleic acid is activated. However, in this case, since the binding and detection of the target nucleic acid and the presence or absence of the reporter nucleic acid activation are performed at different stages, not only is the sensitivity reduced, but also additional steps must be performed, resulting in the overall diagnostic method becoming complicated.

However, in the present invention, the target nucleic acid may be selectively attached to the surface of the microparticle by the CRISPR system as described above, and the reporter nucleic acid is complementarily bound to the target nucleic acid, and therefore, high selectivity may be achieved and sensitivity may also be increased.

In addition, in the diagnostic method using the conventional CRISPR system, the presence or absence of the target nucleic acid was detected by blocking the signal by cleavage of part or all of the reporter nucleic acid when the target nucleic acid is bound as described above, but in this method, if the concentration of the target nucleic acid is low, the signal is blocked by cleavage of only part of the reporter nucleic acid on the surface of the microparticle, so that the intensity of the signal may be somewhat reduced and the signal may still be emitted. As a result, false positives may increase significantly and sensitivity may inevitably be low. However, in the present invention, since the target nucleic acid labeled with the reporter nucleic acid is captured on the surface of the microparticle by the CRISPR system, even if the concentration of the target nucleic acid is low, some of the reporter nucleic acids may generate a signal, and therefore, high sensitivity may be achieved.

The reporter nucleic acid 130 generates a signal, for example, an optical or electrical signal, by external stimuli including chemical, mechanical, optical and electrical energy. In one embodiment, the reporter nucleic acid 130 may be a nucleic acid labeled with a luminescent material and may generate a luminescent signal such as fluorescence or chemiluminescence by external light or a chemical reaction.

Specifically, the reporter nucleic acid 130 may be DNA complementary to the target nucleic acid. In addition, the reporter nucleic acid may be a 15-mer to 40-mer nucleic acid. In addition, it may be 18-mer to 30-mer, 19-mer to 25-mer, or 20-mer to 23-mer.

The luminescent material is linked to the reporter nucleic acid to generate light by external stimuli such as ultraviolet rays, electron beams, chemical reactions and enzyme-substrate reactions, thereby enabling detection of the presence of the target nucleic acid. Examples of the luminescent materials may include fluorescent molecules, quantum dots, metal nanoparticles, magnetic nanoparticles, enzymes, enzymatic substrates, etc. Among these, fluorescent molecules may be selected as various luminescent materials in terms of ease of acquisition and convenience of application.

As examples of the fluorescent molecules, fluorescent molecules selected from the group consisting of fluorescein isothiocyanate (FITC), fluorescein, fluorescein amidite (FAM), phycoerythrin (PE), europium, tetramethyl-rhodamine isothiocyanate (TRITC), Cyanine 3 (Cy3), Cyanine 5 (Cy5), Cyanine 7 (Cy7), Alexa Fluor dye and rhodamine may be commonly used.

To detect various biomarkers in a sample, various fluorophores such as FITC, PE and Alexa-647 may be introduced and labeled at the 5'-position of the reporter nucleic acid 130.

In addition, in the present invention, the fluorescent material may be supplied after the target nucleic acid is bound to the capture probe and may bind to the reporter nucleic acid. In this case, since the fluorescent material that is not bound to the reporter nucleic acid and is non-specifically bound may be simply removed by washing, only the specifically bound target nucleic acid and the fluorescent material remain on the surface of the microparticle.

The multi-diagnostic system may comprise a reagent for nucleic acid amplification. The reagent for nucleic acid amplification is for amplifying a target nucleic acid and is not particularly limited, but may be a loop-mediated isothermal amplification (LAMP), single primer isothermal amplification (SPIA), recombinase polymerase amplification (RPA), etc., in which nucleic acid amplification is performed under isothermal conditions, and may preferably be an RPA reagent in terms of rapidity and low-temperature reaction characteristics. For example, a RT-RPA reagent may contain target-specific primers for specifically amplifying a target nucleic acid. In addition, as discussed above, the reporter nucleic acid may also be amplified while the target nucleic acid is amplified. The reporter nucleic acid of the present invention has a sequence complementary to the target nucleic acid. Therefore, the reporter nucleic acid may also be amplified during the amplification process of the target nucleic acid, and as a result, most of the target nucleic acids amplified as described above may exist bound with the reporter nucleic acids.

The diagnostic microparticle probe may be labeled to be distinguished from each other by diameter, thickness, length, shape or identification code.

In the present invention, genetic scissors such as the CRISPR-Cas system are used to capture a target nucleic acid on the surface of the microparticle. In this case, when two or more types of genetic scissors such as the CRISPR-Cas system are used, it is possible to simultaneously capture different target nucleic acids.

However, since it is difficult to distinguish the types of target nucleic acids with only this simultaneous capture, if the shapes of the microparticles are different and each genetic scissors such as the CRISPR-Cas system is used, different target nucleic acids may be easily distinguished based on the difference in shape, and simultaneous detection is also possible.

For example, if the microparticle probe is a microrod, the type and amount of each nucleic acid may be rapidly and independently obtained through the process of analyzing bright-field images and fluorescence images for a first detection nucleic acid and a second detection nucleic acid, by using microrods of different lengths and applying genetic scissors technology such as the CRISPR-Cas system thereto. For example, if microrods made by cutting glass-coated microwires into various lengths are used as microparticle probes, it is possible to analyze single base polymorphism with just one fluorescent material. By interpreting microparticle probes encoded with each length information through image analysis, it has the advantage of being able to perform multi-detection at once and maintain high-sensitivity accuracy.

Separately from this, it is also possible to distinguish each target nucleic acid using a fluorescent material attached to the reporter nucleic acid. When two or more types of reporter nucleic acids that specifically bind to each fluorescent material are used, the reporter nucleic acids may emit fluorescence with different wavelengths depending on the type of each target nucleic acid, and if they are distinguished through polarization analysis or wavelength analysis, the presence or absence of two or more types of target nucleic acids may be determined simultaneously. However, in this method, the types of reporter nucleic acids combined with fluorescent materials are limited, and in some fluorescent materials, since the emission wavelengths overlap or light sources of various wavelengths are required for emission, the efficiency may be lower than that of the method using the shape of the microparticles.

The present invention also provides a method for detecting a target nucleic acid, comprising the steps of: (a) extracting a target nucleic acid from a sample; (b) amplifying the target nucleic acid and binding a reporter nucleic acid thereto; (c) providing a diagnostic microparticle probe comprising a microparticle; a capture probe introduced to the surface of the microparticle; and an inactivated genetic scissors bound to the capture probe and containing a gene sequence complementary to the target nucleic acid; (d) reacting the diagnostic microparticle probe with the target nucleic acid to capture the target nucleic acid and the reporter nucleic acid; (e) attaching a fluorescent material to the reporter nucleic acid; and (f) detecting the target nucleic acid by measuring a fluorescent signal emitted from the dyed reporter nucleic acid.

First, (a) the target nucleic acid is extracted from the sample. A lysis solution containing guanidinium thiocyanate may be used to release the nucleic acids within the cell by destroying the cell membrane of the sample. Nucleic acids in the solution may be attached to magnetic silica beads or silica-coated microparticles 110 of the present invention, concentrated, washed with alcohol, and detached using an elution buffer.

Next, (b) the target nucleic acid is amplified. For example, the target nucleic acid may be specifically amplified using real-time reverse transcriptase-polymerase chain reaction (RT-RPA) reagents. In addition, as discussed above, during this process, the reporter nucleic acid may also be amplified simultaneously, and after the amplification is completed as described above, the target nucleic acid and the reporter nucleic acid may be complementarily bound.

Subsequently, (c) a diagnostic microparticle probe is provided. The diagnostic microparticle probe comprises a microparticle; a capture probe introduced to the surface of the microparticle; and an inactivated genetic scissors bound to the capture probe and containing a gene sequence complementary to the target nucleic acid. A detailed description of each component of the present microparticles is as described above.

(d) The target nucleic acid and the reporter nucleic acid may be captured by reacting the diagnostic microparticle probe with the target nucleic acid. As discussed above, since the guide RNA included in the genetic scissors in the diagnostic microparticle probe may complementarily bind to the target nucleic acid, the target nucleic acid may be selectively captured by the diagnostic microparticle probe. In this case, the reporter nucleic acid is complementarily bound to the target nucleic acid, and the reporter nucleic acid may also be attached to the diagnostic microparticle probe. As a result, a diagnostic microparticle probe-nucleic acid complex may be formed.

In addition, the formation of the complex may comprise a process of promoting a reaction using an external magnetic force. For example, a well containing reactants may be heated to an appropriate temperature to promote a reaction, and microparticles containing magnetic materials may be controlled by continuously moving a magnetic rod up and down inside the well to promote the reaction.

(e) A fluorescent material may be attached to the reporter nucleic acid. The fluorescent material may be attached to the reporter nucleic acid and may emit fluorescence by an external stimulus in the step described below.

In addition, the fluorescent material should be attached only to the reporter nucleic acid, but it may be non-specifically attached or physically adsorbed to an undesired location. This abnormal attachment of fluorescent materials acts as a factor in indicating false positive results. In the present invention, such abnormally bound fluorescent materials may be easily removed by washing. That is, the method may comprise washing the diagnostic microparticle probe to which the target nucleic acid and reporter nucleic acid are attached, after the step (e).

In addition, this washing may remove reporter nucleic acids not bound to the target nucleic acids and residual reagents from the previous process, thereby increasing the accuracy of diagnosis by the method of the present invention.

The step (f) may be detecting the target nucleic acid by measuring a fluorescent signal emitted from the dyed reporter nucleic acid.

If the target nucleic acid, reporter nucleic acid and fluorescent material are attached to the diagnostic microparticle probe as described above, the fluorescent material may generate a signal through an external stimulus. In this case, the target nucleic acid is detected by measuring the on-off of the signal emitted from the complex by the external stimulus. The signal may be a luminescence signal emitted from a luminescent material within the complex. The external stimuli may be ultraviolet rays, electron beams, chemical reactions, enzyme-substrate reactions, etc. For example, before the target nucleic acid and reporter nucleic acid are bound to the diagnostic microparticle probe as described above, the diagnostic microparticle probe does not emit light even when an external stimulus such as ultraviolet ray is applied. However, if the target nucleic acid and reporter nucleic acid are attached to the guide RNA through the above process and the fluorescent material is bound to the reporter nucleic acid, the diagnostic microparticle probe may emit light by the external stimulus as described above. Therefore, by overlapping and analyzing the fluorescence image with the bright-field image, the microparticle probe complexed with the target nucleic acid may be identified, so that the target nucleic acids may be distinguished.

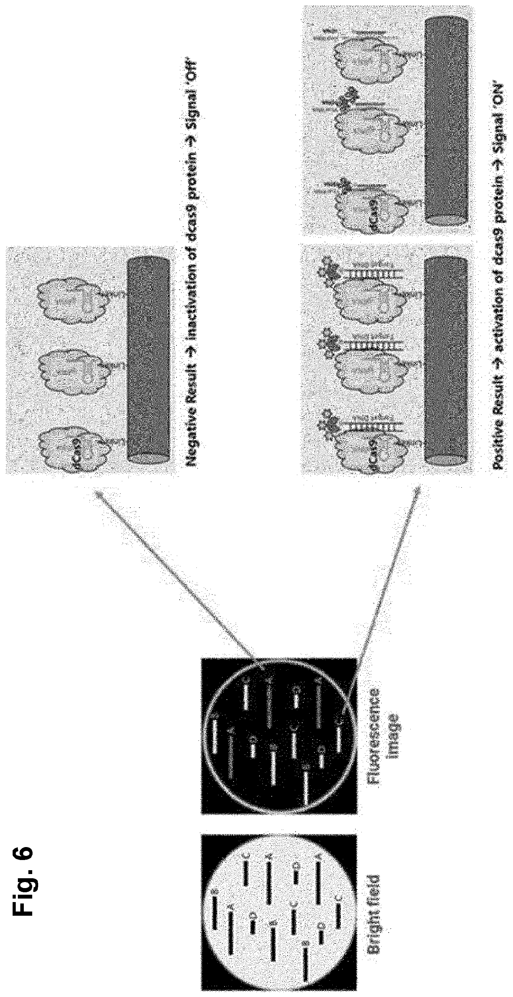

In this case, the detection of the target nucleic acid may be to identify the presence or absence of the target nucleic acid by measuring the difference in luminescence of each probe using microparticle probes for the analysis of two or more single base polymorphisms. That is, since different types of probes emit light when an undesired nucleic acid is present and when a desired target nucleic acid is present, they may be distinguished by taking bright-field images and fluorescence images in parallel. Separately from this, even in cases where two or more types of target nucleic acids exist, they may be individually distinguished and attached to each diagnostic microparticle probe, so that it is possible to distinguish them individually through the shape of the diagnostic microparticle probe on the fluorescence image (see FIG. 6).

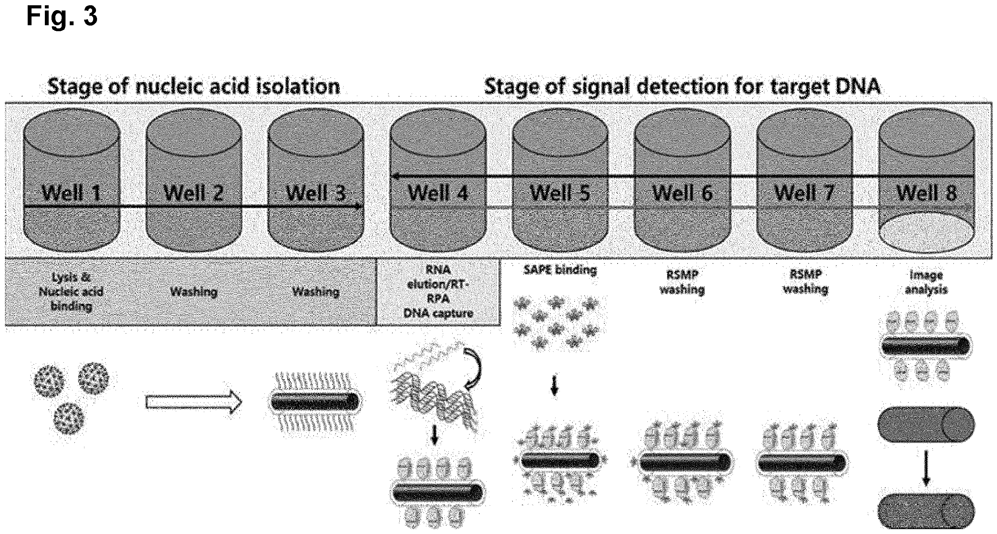

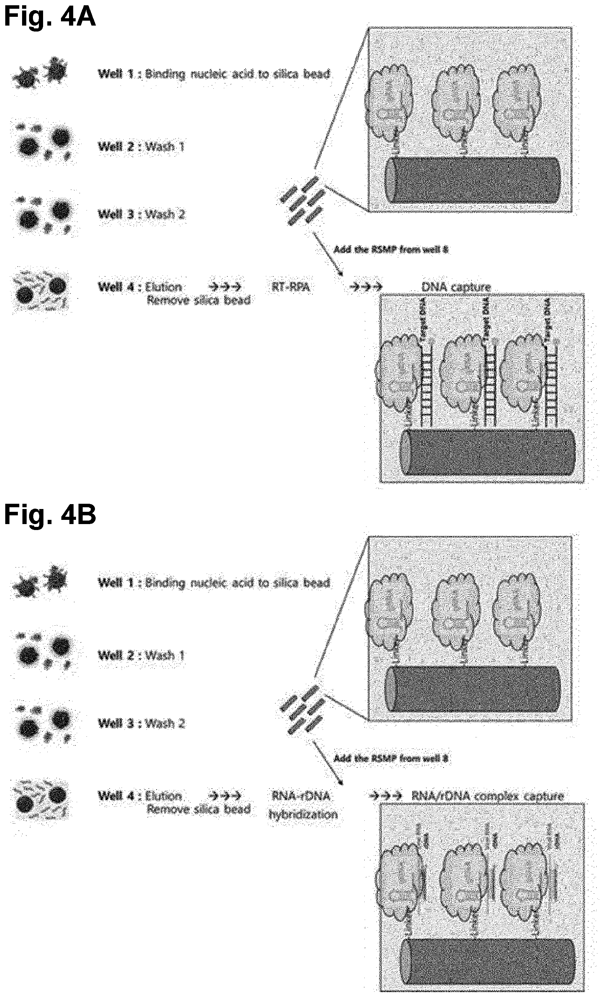

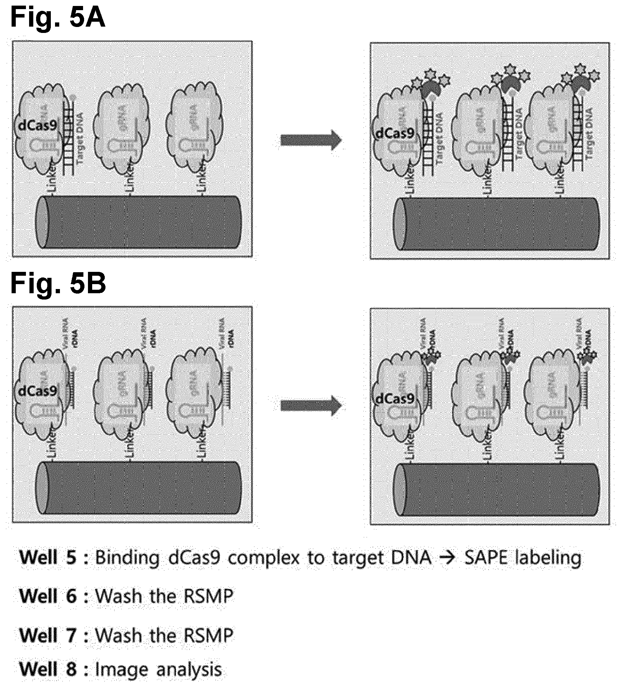

The method for detecting the target nucleic acid may be performed on a microwell. Although it is possible to carry out the detection process as described above in each test tube or container, miniaturization and kitting may be achieved by carrying it out on a microwell. That is, a diagnostic microparticle probe, a buffer, a reagent for amplification, a washing buffer, etc. may be sequentially loaded inside the microwell, and the method for detecting the target nucleic acid may be performed by supplying the sample and sequentially moving the diagnostic microparticle probe (see FIGS. 3 to 5).

The details of this are as follows.

### 1) Well 1: Binding of nucleic acids to microparticles

By placing a sample into Well 1 containing magnetic microparticles, a chaotropic salt and a lysis solution to destroy the membrane of the sample, nucleic acids within the membrane are released, and the nucleic acids are attached to the magnetic microparticles by the action of the chaotropic salt.

### 2) Well 2: Washing and removal of impurities

The magnetic microparticles to which nucleic acids are attached are transferred to Well 2, and unnecessary residual impurities are removed with a solution containing 33% isopropanol and salt.

### 3) Well 3: Washing

The magnetic microparticles to which nucleic acids are bound are washed using 70% ethanol. In this process, salts other than nucleic acids are removed.

### 4) Well 4: Nucleic acid amplification or hybridization

The magnetic microparticles to which nucleic acids are bound are transferred to Well 4 containing a reaction buffer, and the nucleic acids are detached from the magnetic microparticles. Next, the cDNA of the target nucleic acid is synthesized and amplified with the RT-RPA reagent contained in Well 4, or the target nucleic acid is hybridized with a biotin-labeled reporter nucleic acid having a complementary sequence to the target nucleic acid. The RT-RPA reagent may contain target-specific primers for specifically amplifying RSV, influenza and coronavirus genes and labeling them with biotin.

Meanwhile, three types of magnetic microparticle probe/inactivated genetic scissors complexes with three different lengths are prepared. Each magnetic microparticle probe complex of a specific length is immobilized with a guide RNA/dCas9 complex that targets only a specific virus. As the target gene is amplified by RT-RPA, it is captured by the magnetic microparticle complex that recognizes the double-stranded site of each target gene amplified in this process, forming a capture body.

On the other hand, without the RT-RPA process, the biotin-labeled reporter nucleic acid having a complementary sequence to the target nucleic acid is hybridized with the target nucleic acid, and three types of magnetic microparticle complexes with three different lengths are reacted to capture the target gene, forming a capture body.

### 5) Well 5: Fluorescence staining of magnetic microparticle target nucleic acid capture bodies

The magnetic microparticle target nucleic acid capture bodies whose reaction is completed in Well 4 are transferred to well 5 containing streptavidin, R-phycoerythrin conjugate (SAPE). SAPE binds to biotin labeled on the magnetic microparticle target nucleic acid capture bodies.

### 6) Well 6: Washing

The magnetic microparticle target nucleic acid capture bodies whose reaction is completed are washed with a washing buffer.

### 7) Well 7: Washing

The magnetic microparticle target nucleic acid capture bodies whose reaction is completed are washed once more with a washing buffer.

### 8) Well 8: Image analysis

The magnetic microparticle target nucleic acid capture bodies whose reaction and washing are completed are imaged. In this case, both bright-field images and fluorescence images are obtained.

The above-described diagnostic microparticle probe and the diagnostic method using the same may have the following advantages. The present invention has overcome the limitations of POCT analysis by the conventional reverse transcriptase-polymerase chain reaction (RT-PCR) method by using CRISPR-CAS genetic scissors technology. The conventional molecular diagnostic method for POCT analysis consists of the process of sample collection on site, gene extraction, amplification and detection at a contracted institution, and used expensive equipment to obtain the results of gene amplification. These processes were not suitable for mass sample testing on site and took at least six hours to obtain results. However, the present invention enables the process of gene extraction, amplification and detection to be performed onsite and automated by introducing the CRISPR-Cas genetic scissors technology (the time required for the gene extraction and amplification steps may be significantly reduced by use of magnetic particles). In addition, the present invention has overcome the non-specific amplification phenomenon seen in conventional molecular diagnosis by using inactivated CRISPR-Cas genetic scissors technology. Finally, in the diagnostic methods using conventional activated CRISPR-Cas genetic scissors technology, the disappearance of fluorescence is judged as positive, but in this case, there is a high possibility of false positives if the amount of target nucleic acid is small. However, in the present invention, since the emission of fluorescence is judged as positive by using inactivated genetic scissors, it may have higher selectivity and sensitivity than conventional methods.

Hereinafter, preferred examples of the present invention will be described so that those of ordinary skill in the art can easily implement them with reference to the accompanying drawings. In addition, in describing the present invention, if it is determined that the specific description of the related known functions or known configurations may unnecessarily obscure the gist of the present invention, the detailed description thereof will be omitted. In addition, certain features presented in the drawings are enlarged, reduced, or simplified for ease of explanation, and the drawings and components are not necessarily drawn to scale. However, those skilled in the art will readily appreciate these details.

### EXAMPLES

### Example 1. Preparation of magnetic microparticles for capturing nucleic acids

In this POCT multi-diagnostic system, eight wells were used, and in order to move nucleic acids between each well, the use of magnetic microparticles that may stably capture nucleic acids extracted from target factors and move them between wells is essential. To stably capture nucleic acids, magnetic particles for capturing nucleic acids with sufficient surface area were prepared using cylindrical magnetic microparticles or commercially available magnetic microparticles with a size of 10 to 1000 µm as raw materials.

First, the prepared basic magnetic microparticles (prepared by our company, basic core-shell type magnetic microparticles without a capture probe corresponding to 110 of FIG. 2, and having a cylindrical rod shape with a length of 100 to 500 µm) were washed three times with a 0.1 M sodium hydroxide solution and three times with a 100% ethanol solution, and then ultrasonically washed for 2 seconds.

Next, 200 mg of the magnetic microparticles were added to 10 ml of a solution containing ammonia water (composition of 20 ml of 100% ethanol, 3 ml of deionized water and 1 ml of ammonia water), and 2 ml of tetraethylorthosilicate (TEOS) solution was added based on 100 µl of TEOS per 10 mg of magnetic particles. The solution was rotated at 20 rpm for 20 minutes using a rotator device (FINEPCR, Hybridization Incubator Combi-H12) as one reaction, and 2 ml of TEOS solution was added at each reaction, and the reaction was performed a total of 4 times to complete the production of magnetic microparticles. The magnetic particles obtained after the reaction were washed three times with the previously mentioned ammonia water solution and three times with 100% ethanol, and then stored in an ethanol solution.

For the purpose of verifying the nucleic acid capture ability of magnetic microparticles prepared for nucleic acid capture, any sample was prepared and the actual nucleic acid capture ability was verified. A hypothetical virus or harmful bacteria may be assumed as any sample, wherein any *E. coli* was selected as the target to check whether nucleic acid capture was present.

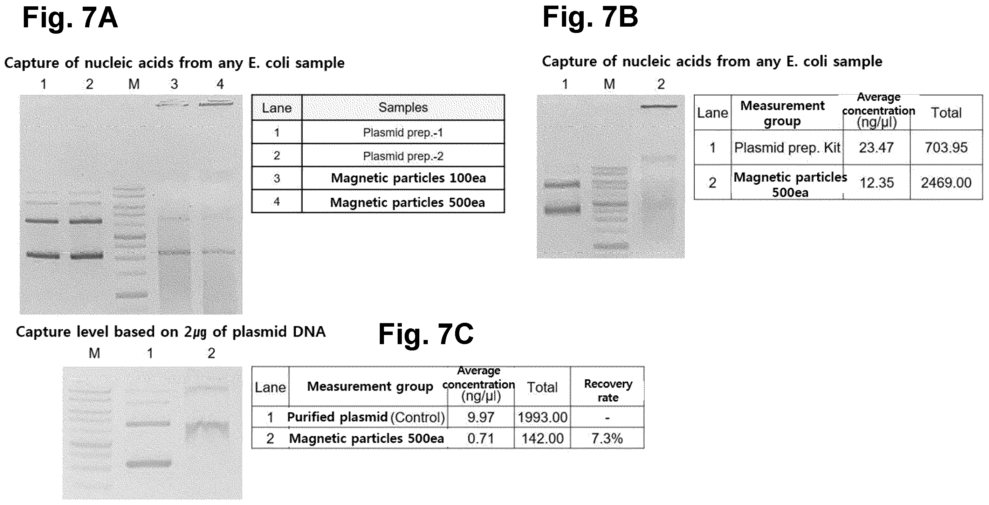

FIG. 7 shows the results of preliminarily confirming the possibility of capturing nucleic acid from a magnetic particle-based target sample. FIG. 7A compares the results of purifying and isolating plasmid DNA from *E. coli* into which plasmid DNA was introduced based on a conventional commercial *E. coli* plasmid mini prep. kit (Bioneer, AccuPrep^{®} Plasmid Mini Extraction Kit) based on an *E. coli* sample set as any target factor, and the results of purifying and isolating plasmid DNA from *E. coli* into which plasmid DNA was introduced by agarose gel-based electrophoresis through magnetic microparticle beads for nucleic acid capture prepared based on our company's magnetic microparticles.

Referring to FIG. 7A, it can be seen that plasmid DNA may be purified and captured under conditions in which 100 and 500 of our company's magnetic particles for nucleic acid purification are present.

Lysis buffer: 1 ml of *E. coli* concentrated sample cultured based on the buffer composition (2 M guanidinium thiocyanate, 36.7 mM Tris-Cl, 16.7 mM EDTA, 2% Triton X-100, 33% isopropanol, RNase free water) constituting Well 1 and our company's magnetic microparticle beads were mixed, and then rotating stirring was performed for 10 minutes; lanes 1 and 2-results 1 and 2 purified based on commercial plasmid prep. kit, lane 3-result purified based on 100 ea of our company's beads for nucleic acid purification, lane 4-result purified based on 500 ea of our company's beads for nucleic acid purification, lane M: 1 kb DNA ladder (Solgent, 1 Kb Plus DNA Ladder).

FIG. 7B shows the results of confirming nucleic acid purification based on the plasmid prep. kit and 500 ea of our company's beads for nucleic acid purification under the same conditions as FIG. 7A. Referring to FIG. 7B, it can be confirmed that both genomic DNA of *E. coli* and plasmid DNA may be purified and recovered through nucleic acid purification based on our company's magnetic particle compared to the plasmid prep. kit.

FIG. 7C shows the results of confirming the capture ability of our company's magnetic particles targeting plasmid DNA at a concentration of 2 µg. Referring to FIG. 7C, the capture ability for any nucleic acid can be confirmed by the fact that the nucleic acid sample may be captured at a rate of about 7.3% through our company's magnetic particles for nucleic acid purification.

### Example 2. Target factor lysis and nucleic acid capture amplification step (Well 1 - Well 4)

The feasibility of applying our company's automated equipment to perform the process from lysis of the target sample to imaging of the magnetic particle probe as a single process was confirmed. A mobility test was conducted to confirm whether the target nucleic acid obtained by lysis and capture was sequentially processed through normal movement between wells.

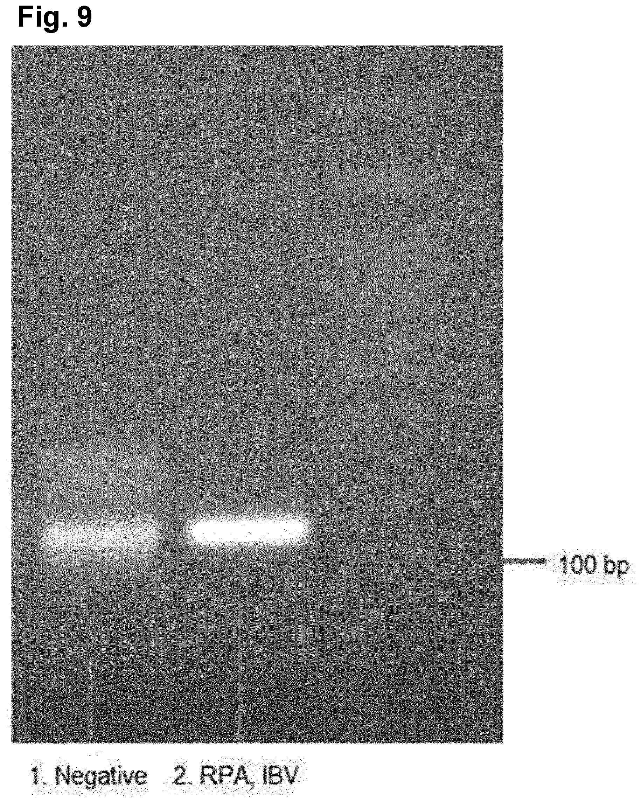

FIG. 9 shows the results of confirming nucleic acid capture of a target sample and nucleic acid amplification of a target site in the multi-diagnostic method according to one embodiment of the present invention.

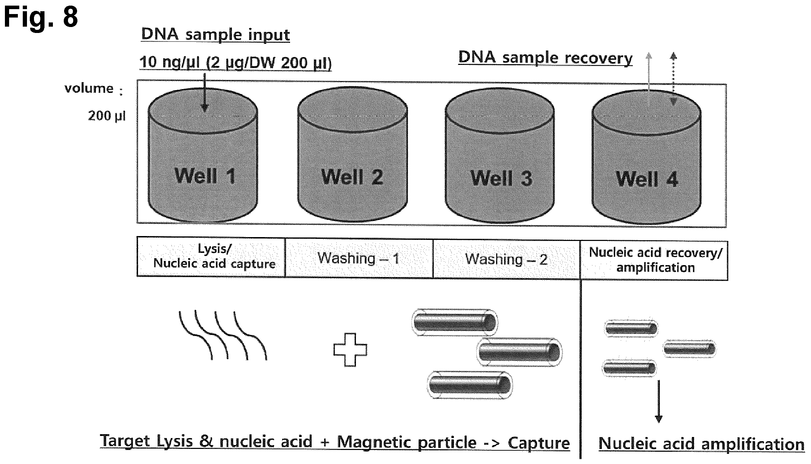

FIG. 8 shows a capture and amplification process of a target nucleic acid sequentially performed from Well 1 to Well 4. Referring to FIG. 8, lysis/nucleic acid capture was performed in Well 1, and nucleic acids were extracted and captured from virus samples using a solution containing chaotropic salts (2 M guanidinium thiocyanate, 36.7 mM Tris-Cl, 16.7 mM EDTA, 2% Triton X-100, 33% isopropanol, RNase free water) for extraction and capture from virus samples.

The first washing process (washing-1) was performed in Well 2, wherein the magnetic particles comprising the captured nucleic acids were washed using 2 M guanidinium thiocyanate, 36.7 mM Tris-Cl, 16.7 mM EDTA, 2% Triton X-100, RNase free water + 66% Isopropanol, and the captured nucleic acids were further concentrated on the magnetic particles.

The second washing process (washing-2) was performed in Well 3, wherein the remaining waste attached to the magnetic particles was removed using 70% ethanol.

Recovery and amplification of nucleic acids were performed in Well 4, wherein the nucleic acids attached to the magnetic particles were detached, and then the detached nucleic acids were amplified. A solution based on the recombinase polymerase amplification (RPA) enzyme complex was used for nucleic acid amplification.

FIG. 9 shows the result of performing a capture and amplification process of a nucleic acid for RNA of IBV, which is a type of influenza. In Well 4, the detached target nucleic acid was subjected to reverse transcriptase recombinase polymerase amplification (RT-RPA) reaction at 37° C for 20 minutes under conditions in which IBV RNA was added. The composition of the RPA reagent used in this case was subjected to the reaction according to the protocol recommended by the manufacturer (TwistDx, TwistAmp Liquid Kit). Referring to FIG. 9, it can be confirmed that after the RNA sample was captured and moved between the wells, the nucleic acid was dissociated in Well 4, and then nucleic acid amplification was performed normally by the RT-RPA enzyme complex.

Table 1 below shows primers for target sequence amplification.

**[Table 1]**

| COVID19 RPA | |
|---|---|
| Forward | 5'-/Biotin-TEG/ATAATGGACCCCAAAATCAGCCGAATGCACCCC/-3' (SEQ ID NO: 1) |
| Reverse | 5'- GTGAGAGCGGTGAACCAAGACGCAGTATTATTG-3' (SEQ ID NO: 2) |

| Influenza A RPA | |
|---|---|
| Forward | 5'-/Biotin-TEG/GAACTATTACTGGACACTAGTAGAGCCTGGAGAC-3' (SEQ ID NO: 3) |
| Reverse | 5'-CGTGGACTGATGTATCTGAAATGATGATACCAG-3' (SEQ ID NO: 4) |

| Influenza B RPA | |
|---|---|
| Forward | 5'-/Biotin-TEG/5'-ATGTCGCTGTTTGGAGACACAATTGCCTACTTG-3' (SEQ ID NO: 5) |
| Reverse | 5'-GTTTTTTATCCATTCCAAGGCTGAGTCTAGGTC-3' (SEQ ID NO: 6) |

Based on the results, it could be indirectly confirmed that target signal detection and POCT diagnosis based on amplified nucleic acids are possible.

### Example 3. Construction and functional verification of magnetic particle probes for signal detection (Well 4 - Well 5)

In the magnetic particle probe for signal detection, dCas9 is coupled to the surface of the magnetic particle, and the magnetic particle probe is constructed in the form of a complex of guide RNA (gRNA) and dCas9, which plays a role in recognizing the target site sequence of the target factor.

The inactivated genetic scissors (dCas9) used in this case were Alt-R S.p. dCas9 Protein V3 purchased from IDT.

When nucleic acids are amplified through RT-RPA in Well 4, the magnetic particle probe for signal detection in Well 7 is transferred to Well 4, wherein the amplified nucleic acids are captured by the gRNA/dCas9 complex.

The magnetic microparticle target nucleic acid capture bodies whose reaction is completed in Well 4 are transferred to well 5 containing streptavidin, R-phycoerythrin conjugate (SAPE). SAPE binds to biotin labeled on the magnetic microparticle target nucleic acid capture bodies.

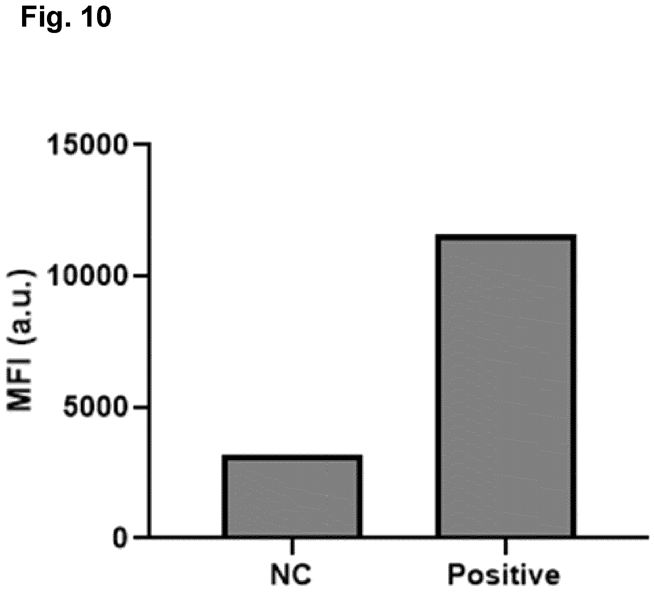

FIG. 10 shows the result of confirming Cas system activity to check whether the fluorescence intensity changes depending on the presence or absence of target nucleic acid by the magnetic particle probe when target nucleic acid is present. In an experiment conducted to confirm the concept of the Cas system, it could be confirmed that the biotin-labeled target nucleic acid was captured by the gRNA/dCas9 complex of the magnetic particle probe and a fluorescent signal was detected.

Table 2 below shows the information on guide RNA.

**[Table 2]**

| COVID19 |
|---|
| 5'-**AAA** CGU AAU GCG GGG UGC AUG UUU UAG AGC UAG AAA UAG CAA GUU |
| AAA AUA AGG CUA GUC CGU UAU CAA CUU GAA AAA GUG GCA CCG AGU CGG UGC **UUU** U-3' (SEQ ID NO: 7) Bold base sequences are 2'-o-methyl phosphorothioate. |

| Influenza A |
|---|
| 5'-**UUC** GAA UGU UAU CUU GUC UCG UUU UAG AGC UAG AAA UAG CAA GUU AAA AUA AGG CUA GUC CGU UAU CAA CUU GAA AAA GUG GCA CCG AGU CGG UGC **UUU** U-3' (SEQ ID NO: 8) Bold base sequences are 2'-o-methyl phosphorothioate. |

| Influenza B |
|---|
| 5'-**UUC** UAU CAA UGA AAG CAA GUG UUU UAG AGC UAG AAA UAG CAA GUU AAA AUA AGG CUA GUC CGU UAU CAA CUU GAA AAA GUG GCA CCG AGU CGG UGC **UUU** U-3' (SEQ ID NO: 9) Bold base sequences are 2'-o-methyl phosphorothioate. |

### Example 4. Confirmation of virus detection by magnetic particle probes for signal detection

Based on the previous examples that confirmed the possibility of detecting a target factor, it was confirmed whether a single target factor could be diagnosed.

To confirm signal detection for a single target factor, three types of respiratory virus-targeting magnetic particle probes were constructed, and signal detection results for influenza A, influenza B and COVID19 virus targets were confirmed.

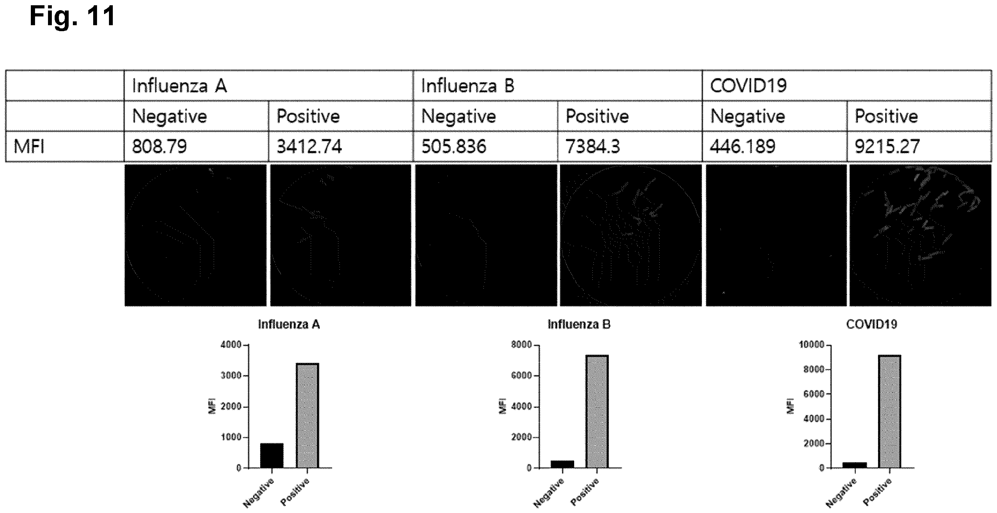

FIG. 11 shows results of confirming that influenza A, influenza B and COVID19 viruses may be detected using the corresponding magnetic particle probes for signal detection.

It was confirmed that the fluorescent signal intensity value of the corresponding magnetic particle probe group for signal detection (3412.74) in which the influenza A target is present was remarkably high compared to the fluorescent signal intensity value of the negative control (808.79) in which the target nucleic acid is not present.

In addition, in an experiment using a magnetic particle probe for influenza B detection, it was confirmed that the fluorescent signal intensity value for influenza B detection was 7384.3 compared to the fluorescent signal intensity value of the negative control (505.836), and in an experiment using a magnetic particle probe for COVID19 detection, it was confirmed that the fluorescent signal intensity value for COVID19 detection was 9215.27 compared to the fluorescent signal intensity value of the negative control (446.189). Therefore, it could be confirmed that when each respiratory virus is present, the RNA of the corresponding virus is extracted and amplified, and captured by a magnetic particle probe for signal detection with the corresponding length, and a fluorescent signal is detected.

### Example 5. Verification of multi-detection of magnetic particle probes for signal detection

Based on the previous example in which it was confirmed that each respiratory virus could be detected, it was confirmed whether multi-detection was possible for three types of respiratory viruses.

A magnetic particle probe with a length of 200 um is for detecting influenza A, a magnetic particle probe with a length of 300 um is for detecting influenza B, and a magnetic particle probe with a length of 400 um is for detecting COVID19. An experiment was conducted to verify that multi-diagnosis is possible when all three magnetic particle probes with different lengths are present.

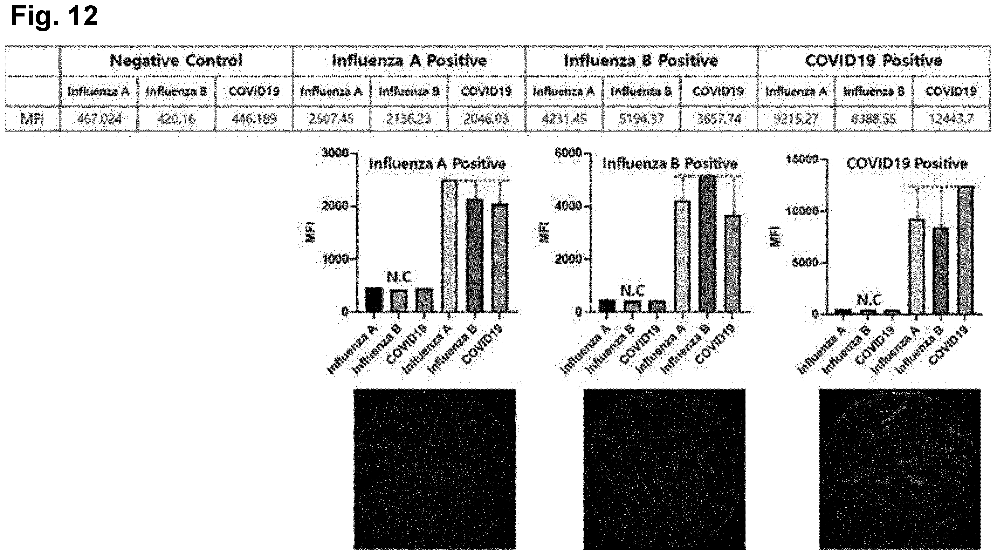

For the Influenza A Positive of FIG. 12, an experiment was conducted to specifically detect the influenza A target in an environment where three magnetic particle probes with different lengths were present. It was confirmed that the fluorescent signal intensity values of the influenza B-targeting magnetic particle probe (2136.23) and the COVID19-targeting magnetic particle probe (2046.03) were low compared to the fluorescent signal intensity value of the influenza A-targeting magnetic particle probe (2507.45).

For the Influenza B Positive of FIG. 12, an experiment was conducted to specifically detect the influenza B target in an environment where three magnetic particle probes with different lengths were present. It was confirmed that the fluorescent signal intensity values of the influenza A-targeting magnetic particle probe (4231.45) and the COVID19-targeting magnetic particle probe (3657.74) were low compared to the fluorescent signal intensity value of the influenza B-targeting magnetic particle probe (5194.37).

For the COVID19 Positive of FIG. 12, an experiment was conducted to specifically detect the COVID19 target in an environment where three magnetic particle probes with different lengths were present. It was confirmed that the fluorescent signal intensity values of the influenza A-targeting magnetic particle probe (9215.27) and the influenza B-targeting magnetic particle probe (8388.55) were low compared to the fluorescent signal intensity value of the COVID19-targeting magnetic particle probe (12443.7). Therefore, it could be confirmed from the graph analysis results in FIG. 12 that they may recognize each target and act as a magnetic particle probe for each when each target is present and when all targets are present.

As a specific part of the present invention has been described in detail above, it will be apparent to those skilled in the art that these specific descriptions are only preferred embodiments, and the scope of the present invention is not limited thereby. Accordingly, it is intended that the substantial scope of the present invention is defined by the appended claims and equivalents thereof.

## Claims

1. A diagnostic microparticle probe, comprising:
a microparticle;
a capture probe introduced to the surface of the microparticle; and
an inactivated genetic scissors bound to the capture probe and containing a genetic sequence complementary to a target nucleic acid.

2. The diagnostic microparticle probe according to claim 1, wherein the inactivated genetic scissors comprise a guide RNA.

3. The diagnostic microparticle probe according to claim 2, wherein the guide RNA has a nucleotide sequence capable of hybridizing with a target site of the target nucleic acid gene.

4. The diagnostic microparticle probe according to claim 1, wherein the inactivated genetic scissors have an inactivated target nucleic acid cleavage function.

5. The diagnostic microparticle probe according to claim 1, wherein the microparticle contains magnetic particles therein.

6. The diagnostic microparticle probe according to claim 1, wherein the microparticle has a core-shell structure comprising a core containing a magnetic material and a shell layer surrounding the core and having a uniform thickness.

7. The diagnostic microparticle probe according to claim 1, wherein the microparticle has a size and specific gravity that prevents it from floating in water.

8. A POCT multi-diagnostic system, comprising:
the diagnostic microparticle probe according to any one of claims 1 to 7;
a reporter nucleic acid complementarily bound to a target nucleic acid and labeled with biotin; and
a reagent for nucleic acid amplification.

9. The POCT multi-diagnostic system according to claim 8, wherein the diagnostic microparticle probe is labeled to be distinguished from each other by diameter, thickness, length, shape or identification code.

10. The POCT multi-diagnostic system according to claim 9, wherein the diagnostic microparticle probe is a mixture of two or more diagnostic microparticle probes with different lengths.

11. The POCT multi-diagnostic system according to claim 8, wherein the reporter nucleic acid is a mixture of two or more reporter nucleic acids to which phosphors with different colors are bound.

12. A method for detecting a target nucleic acid, comprising the steps of:
(a) extracting a target nucleic acid from a sample;
(b) amplifying the target nucleic acid and binding a reporter nucleic acid thereto;
(c) providing a diagnostic microparticle probe comprising a microparticle; a capture probe introduced to the surface of the microparticle; and an inactivated genetic scissors bound to the capture probe and containing a gene sequence complementary to the target nucleic acid;
(d) reacting the diagnostic microparticle probe with the target nucleic acid to capture the target nucleic acid and the reporter nucleic acid;
(e) attaching a fluorescent material to the reporter nucleic acid;
(f) detecting the target nucleic acid by measuring a fluorescent signal emitted from the dyed reporter nucleic acid.

13. The method for detecting a target nucleic acid according to claim 12, wherein the detecting of the target nucleic acid is multi-detecting two or more of the target nucleic acids using two or more of the diagnostic microparticle probes.

14. The method for detecting a target nucleic acid according to claim 13, wherein the multi-detecting comprises the process of reading the diagnostic microparticle probes labeled to be distinguished from each other by length, diameter, thickness, shape, color or identification code.

15. The method for detecting a target nucleic acid according to claim 12, further comprising washing the diagnostic microparticle probe to which the target nucleic acid and reporter nucleic acid are attached, after the step (e).

16. The method for detecting a target nucleic acid according to claim 12, wherein the method for detecting the target nucleic acid is performed on a microwell.

17. The method for detecting a target nucleic acid according to claim 16, wherein the diagnostic microparticle probe is moved and immersed between the microwells by an external magnetic force.
